(19) Europäisches Patentamt
European Patent Office
Office européen des brevets

(11) EP 2 835 630 A1

(12) EUROPEAN PATENT APPLICATION

(43) Date of publication:
11.02.2015 Bulletin 2015/07

(51) Int Cl.:
*G01N 21/77* (2006.01)　　*C12Q 1/68* (2006.01)

(21) Application number: 13003900.1

(22) Date of filing: 05.08.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Max-Planck-Gesellschaft zur
Förderung
der Wissenschaften e.V.
80539 München (DE)

(72) Inventor: The designation of the inventor has not
yet been filed

(74) Representative: Hertz, Oliver
v. Bezold & Partner
Patentanwälte
Akademiestrasse 7
80799 München (DE)

(54) **Sensor device and method for label-free detection of nucleic acid sequences**

(57)　　A sensor device (100), which is adapted for detecting target molecules having a target nucleic acid sequence, comprises an optical whispering gallery mode (WGM) resonator (10) having a resonance frequency, wherein the WGM resonator is functionalized with a double-strand DNA precursor compound and the resonance frequency depends on a mass load provided by the double-strand precursor compound, the double-strand precursor compound is capable of a target-specific strand displacement reaction with the target molecules, and in response to the strand displacement reaction, the double-strand precursor compound is capable to be partially decoupled from the WGM resonator (10), wherein the mass load can be decreased and the resonance frequency of the WGM resonator can be increased. Furthermore, a sensing method for detecting target molecules including nucleic acid sequences is described.

FIG. 1

**Description**

Field of the invention

The present invention relates to a sensor device for label-free detecting nucleic acid sequences, comprising an optical whispering gallery mode (WGM) resonator. Furthermore, the present invention relates to a sensing method for label-free detecting nucleic acid sequences using the WGM resonator. Applications of the invention are available e. g. in the fields of research and clinical nucleic acid sequence detection or screening.

Technical background of the invention

In the present specification, reference is made to the following publications cited for illustrating prior art techniques, in particular relating to conventional detection of nucleic acids and an applications of strand displacement reactions for detecting purposes.

[1] Vollmer, F. et al., Nanophotonics 1, 267-291 (2012),
[2] Fan, X. D. et al., Anal. Chim. Acta 620, 8-26 (2008),
[3] Qavi, A. J. et al., Anal. Bioanal. Chem. 394, 121-135 (2009),
[4] Hunt, H. K. et al., Nanoscale 2, 1544-1559 (2010),
[5] Vollmer, F. et al., Nat. Methods 5, 591-596 (2008),
[6] Yoshie, T. et al., Sensors 11, 1972-1991 (2011),
[7] Lin, S. Y. et al., Lab Chip 11, 4047-4051 (2011),
[8] Lu, T. et al., Proc. Natl. Acad. Sci. U. S. A. 108, 5976-5979 (2011),
[9] Lopez-Yglesias, X. et al., J. Appl. Phys, 111 (2012),
[10] Vollmer, F. et al., Proc. Natl. Acad. Sci. U. S. A. 105, 20701-20704 (2008),
[11] Qavi, A. J. et al., Angew. Chem.-Int. Edit. 49, 4608-4611 (2010),
[12] Nakatani, K. et al., Chembiochem 5, 1623-1633 (2004),
[13] Vollmer, F. et al., Biophys. J. 85, 1974-1979 (2003),
[14] Qavi, A. J. et al., Anal. Chem. 83, 6827-6833 (2011),
[15] Suter, J. D. et al. et al., Biosens. Bioelectron. 23, 1003-1009 (2008),
[16] Zhu, J. G. et al., Nat. Photonics 4, 46 (2010),
[17] Scheler, O. et al., Biosens. Bioelectron. 36, 56-61 (2012),
[18] Zhang, D. Y. et al., Nat. Chem. 4, 208-214 (2012),
[19] Yin, P. et al., Nature 451, 318-U314 (2008),
[20] Li, B. L. et al., Nucleic Acids Res. 39 (2011),
[21] Zhang, D. Y. et al., Science 318, 1121-1125 (2007),
[22] Zhang, D.Y. et al., Nat. Chem. 3, 103-113 (2011),
[23] Zhang, D.Y. et al., J. Am. Chem. Soc. 131, 17303-17314 (2009),
[24] Baaske, M. et al., ChemPhysChem 13, 427-436 (2012),
[25] Arnold, S. et al., Optics Letters 28, 272-274 (2003),
[26] Suter, J.D. et al., Biosens. Bioelectron. 26, 1016-1020 (2010),
[27] Lee, M. et al., Anal. Biochem. 282, 142-146 (2000),
[28] Zuker, M., Nucleic Acids Res. 31, 3406-3415 (2003), and
[29] Zhang, D. Y. et al., Nucleic Acids Res. 38, 4182-4197 (2010).

It is generally known that specific detection of nucleic acids, like DNA and RNA, is an important research and clinical goal as nucleic acids act to encode and regulate the expression of genes. Conventional detection techniques are based on detecting label or marker substances, or they use label-free sensors.
Label-based sensors use e. g. fluorescence-based assays to localize and quantitate nucleic acid molecules of interest. However, functionalizing oligonucleotides with fluorescent labels is typically a complex and expensive process that often skews physical and chemical properties, in turn affecting quantitative readout. Label-free sensors circumvent the need for fluorescence modifications, and they are based on e. g. detecting plasmon resonance, electrochemical conductance or mechanical resonance. However, these techniques may have disadvantages in terms of limited sensitivity or specificity, e. g. due to limited kinetics and thermodynamics of direct hybridization at stringent conditions.
A promising label-free sensor comprises an optical whispering gallery mode (WGM) resonator ([1] - [6]). WGM resonators are micron scale optical cavities, such as glass microspheres, capable of confining light by total internal reflection in a small modal volume and only at specific resonance frequencies (resonance wavelengths). These tiny optical resonators exhibit ultra-narrow linewidth, associated with very high quality Q factor, and are extremely sensitive to the binding of biomolecules to the microcavity resonator surface. The changes in permittivity upon binding of analyte result in a shift of the resonance frequency. The high Q factor enables the precise monitoring of small resonance frequency shifts, a method known as the reactive biosensing principle ([1]). With the conventional WGM resonator, the resonance frequency is decreased depending on an increasing mass load in response to a specific binding reaction with a target molecule under investigation. The target molecule can be detected by monitoring the negative frequency shift. Optical WGM sensors are emerging as one of the most versatile and sensitive label-free detecting techniques, providing various mechanisms for sensing, sizing, trapping, and manipulation down to the nanoscale ([1], [7] - [9]).

Advantageously, WGM sensors are simple to fabricate, can be functionalized as well as multiplexed, and are made from inexpensive optical fibers ([2], [4], [6], [10], [11]. However, as a general disadvantage, sequence-specific detection by direct DNA hybridization on WGM sensor devices, faces three important challenges ([2], [11], [12] - [15]): limited sensitivity, specificity, and reusability.

First, years of work on advancing the device physics and engineering of WGM sensors has improved the ultimate physical detection limits of WGM transducers ([1], [8] - [10], [16]), yet the limits for DNA detection by hybridization has plateaued ([11], [13] - [15], [17]). Novel molecular approaches are needed to overcome those limitations, mostly set by the inherent kinetics and thermodynamics of the process of molecular recognition through direct hybridization at the sensor surface.

Second, label-free sensors based on hybridization struggle with single base specificity and SNP detection, due to the thermodynamic favorability of hybridization of non-cognate analytes with highly similar sequence to that of the analyte. Although specificity for any particular nucleic acid analyte/probe pair can be optimized by solution salinity and temperature, this process is time consuming and imperfect, and not conducive to significant multiplexing. Similarly, the suppression of nonspecific interactions is essential to multiplexed detection. Thus, non-cognate sequences that differ slightly in sequence may bind non-specifically to the functionalized surface of conventional sensors, generating false positive signals and preventing proper detection.

Finally, conventional label-free nucleic acid detection technologies based on hybridization suffer from the limitation that a different functionalized device is needed to detect each different sequence. Furthermore, each device can generally be only used once; dehybridizing oligonucleotides requires harsh buffer conditions, high temperature, or practically takes too long (see [27]). Thus, different sensors must be constructed to detect different nucleic acid sequences.

DNA strand displacement techniques have recently emerged as a novel family of approaches to enzyme-free homogenous detection assays ([18] - [21]). Strand displacement circuits, for example, have been demonstrated to implement nucleic acid "catalysis" in which a nucleic acid sequence of interest effects the release of up to 100 nucleic acid molecules from metastable precursors; cascading such catalytic systems ([21]) has shown overall turnover of about 1000. Recently, strand displacement has been engineered to allow ultraspecific hybridization assays with specificity approaching the theoretical limit based on thermodynamics ([18]). Although strand displacement techniques have significantly improved the specificity and sensitivity of homogeneous detection assays, the readout for this technology has previously been constrained to gel electrophoresis or fluorescence readout, neither of which is easily applicable to point-of-care or clinical diagnostics ([18], [21] - [23]).

Objective of the invention

The objective of the invention is to provide an improved sensor device for detecting nucleic acid sequences avoiding limitations of conventional techniques. In particular, the objective of the invention is to provide a sensor device having an increased specificity, sensitivity and/or reusability. Furthermore, the objective is to provide an improved sensing method for detecting nucleic acid sequences avoiding limitations of conventional techniques.

Brief summary of the invention

According to a first general aspect of the invention, the above objective is solved by a sensor device, which is adapted for detecting (sensing) target molecules having a target nucleic acid sequence, comprising an optical whispering gallery mode (WGM) resonator with a functionalized resonator surface which is adapted for a target-specific strand displacement reaction. The WGM resonator is a compact, mirror-free resonator accommodating a circulating light field having a resonance frequency, such as a microdisc, microsphere, microtoroid or microring. The WGM resonator is functionalized with a precursor compound, which can be partially unloaded by the target-specific strand displacement reaction. Unloading of the functionalized WGM resonator surface results in a reduction of the effective resonator size, e. g. diameter, associated with an increasing resonance frequency (decreasing resonance wavelength). Thus, according to the invention, the WGM resonator is adapted for providing an increasing resonance frequency in response to a mass load reduction resulting from the strand displacement reaction of the target molecule with the precursor compound. The target molecule can be detected by monitoring a positive resonance frequency shift. Preferably, the precursor compound is a double-strand DNA precursor compound which is capable of the strand displacement reaction with the target molecules. In response to the strand displacement reaction, the double-strand precursor compound is capable to be partially decoupled from the WGM resonator, so that the mass load can be decreased and the resonance frequency of the WGM resonator can be increased.

According to a second general aspect of the invention, the above objective is solved by a sensing method for detecting target molecules having nucleic acid sequences, wherein a sample liquid to be investigated is applied to a WGM resonator of the sensor device according to the above first aspect of the invention, a resonance frequency of the WGM resonator is measured and the target molecules are detected in the sample liquid if the resonance frequency of the WGM resonator is increased in response to the contact of the sample liquid with the WGM resonator. The target molecule to be detected comprises any molecule (biomolecules) which includes at least one nucleic acid sequence and which is capable of a hybridization reaction with a nucleic acid strand. The target molecule consists of one single nucleic acid sequence to be detected (target nucleic acid sequence), or it has multiple nucleic acid sequences including the target nucleic acid sequence.

Advantageously, the invention provides a nucleic acid detection with a label-free sensor which circumvents costly fluorophore functionalization steps associated with conventional assays by utilizing impressive ultimate detection limits provided by the WGM resonator. Despite this technological progress, molecular recognition at the WGM resonator surface is based on partial unloading of the precursor compound. The inventors have found that the concept of a DNA strand displacement reaction can be applied with a WGM sensor. Contrary to conventional WGM resonator based sensors, the target molecule is not bound to the resonator, but used for unloading the surface functionalization precursor molecules. This allows overcoming limits as to the sensors' sensitivity, specificity, and reusability.

With the invention, the advantages of WGM resonator label-free readouts and DNA strand displacement circuits are combined by constructing an integrated label-free sensor. The integrated sensor is highly specific, able to distinguish single nucleotide polymorphisms (SNPs) via kinetics of mass loading/unloading by a factor of 32 to 690.

Advantageously, various types of strand displacement reactions can be used with the invention. According to a first variant, a single replacement scheme is used wherein each target molecule to be detected is capable of releasing one strand from one precursor compound molecule. This variant has advantages in terms of a simple replacement reaction. According to a second, preferred variant, a catalytic replacement scheme is used wherein each target molecule to be detected is capable of releasing multiple strands from multiple precursor compound molecules. With this variant, a cyclic catalytic reaction pathway is obtained and the sensitivity of the detection can be essentially increased.

With the single replacement scheme of the first variant, the double-strand precursor compound has a primary strand and a secondary strand being hybridized with the primary strand, wherein the secondary strand is connected with the WGM resonator. The double-strand precursor compound can be partially decoupled from the WGM resonator by replacing the secondary strand by the target nucleic acid sequence and dissociating the primary strand hybridized with the target nucleic acid sequence from the WGM resonator. In other words, the double-strand precursor compound is selected such that the target nucleic acid sequence is capable of the replacement hybridization reaction with the, primary strand. The replacement hybridization is energetically preferred compared with the initial hybridization of the primary and secondary strands. Thus, the target nucleic acid sequence specifically replaces the secondary strand. As the primary strand is connected to the WGM resonator via the secondary strand only, this replacement releases the primary strand from the WGM resonator, resulting in the mass load reduction to be obtained.

With the catalytic replacement scheme, the double-strand precursor compound has a primary strand and first and secondary strands being hybridized with the primary strand, wherein the first secondary strand is connected with the WGM resonator. The double-strand precursor compound can be partially decoupled from the WGM resonator by replacing the second secondary strand by the target nucleic acid sequence and by replacing the first secondary strand by a further single-strand precursor molecule (so called fuel strand), so that the primary strand hybridized with the target nucleic acid sequence and the single-strand precursor molecule is dissociated (separated) from the WGM resonator.

Advantageously, this integrated sensor adapted for the catalytic replacement scheme exhibits at least 25-fold improvement in molecular sensitivity over the conventional hybridization-based WGM nucleic acid sensor, thus allowing a detection of 80 pM (32 fmol) of a 22 oligomer. This improvement results from both the catalytic behavior of the functionalized WGM resonator and a decoupling of mass loading from the analyte. Furthermore, the integrated sensor exhibits extremely high specificity, discriminating single nucleotide polymorphisms by a factor of 32 to 690.

According to a particularly preferred embodiment of the catalytic replacement scheme, the single-strand precursor molecule is capable to replace the target nucleic acid sequence from the primary strand, so that the target molecule is directly released from the primary strand into the sample liquid to be tested. Thus, the repeated use of the target molecule for unloading primary strands from the WGM resonator is possible.

According to a preferred embodiment of the invention, the WGM resonator has a surface layer made of a biotin streptavidin compound to which the double-strand precursor compound, in particular the secondary strand (single replacement scheme) or the first secondary strand (catalytic replacement scheme) is coupled.

According to a further advantageous embodiment of the invention, the WGM resonator is provided in contact with at least one optical waveguide or optical prism. One single optical waveguide or prism can be arranged for both of in-coupling sample light (excitation light) into the WGM resonator and out-coupling resonator light out of the WGM resonator. Alternatively, a first optical waveguide or prism can be arranged for in-coupling the sample light, while a second optical waveguide or prism can be arranged for out-coupling the resonator light. Preferably, the at least one optical waveguide or optical prism is connected with the resonator surface.

The sample light (test light) is preferably generated with a continuous wave tunable laser source. The laser source is tuned to the resonance frequency of the WGM resonator loaded with the double-strand precursor compound. The resonator light is the light field resonantly circulating within the WGM resonator. If the target molecule to be detected is present in a sample liquid and the double-strand precursor compound is partially dissociated from the WGM resonator, the resonance frequency of the resonator light is increased compared with the frequency of the sample light. This positive frequency shift generally can be detected e. g. by spectrally resolved measuring the resonator light and comparing it with the spectrum of the sample light.

According to a further advantageous embodiment of the invention, the sensor device further comprises a sample cell being adapted for arranging the WGM resonator and accommodating the sample liquid to be investigated. Preferably, optical waveguides are arranged for carrying the sample light and the resonator light to and from the WGM resonator, resp., in the sample cell. The sample cell may comprise a substrate carrying a frame, like an O ring, accommodating a droplet of the sample liquid, wherein the WGM resonator can be arranged above the frame so that the WGM resonator contacts the sample liquid. Alternatively, the sample cell may comprise a container (vessel) enclosed with container walls. With this embodiment, the optical waveguides pass through the container walls. Using the container cell may have advantages in terms of providing a compact sensor structure which is adapted for point-of-care-uses.

In summary, for real-time, label-free nucleic acid detection, the inventive sensor device provides three major advantages over conventional label-free biosensor approaches: molecular sensitivity, molecular specificity, and device reusability. Experimentally, at least 25-fold enhancement of the sensitivity and detection down to about 80 pM (32 fmol) of a 22-mer DNA oligo, SNP discrimination by a factor of 32 to 690, and versatile detection by the same physical microsphere of 2 different analytes over 5 cycles of use have been shown. Discrimination against SNP variants of the intended analyte has been found to be robust to position and base identity of the SNP.

The versatility and reusability of the inventive sensor device show that the invention is suitable not only for laboratory investigations but also for real-world diagnostic applications. By allowing the same type of DNA-functionalized microsphere to be generally used for the detection of any nucleic acid biomarker, device manufacturing costs are sharply reduced. By allowing the same physical microsphere device to be used across multiple cycles of operation, the number of devices needed by the end-users is reduced. Consequently, the application of inventive sensor device to point-of-care diagnostics is facilitated.

Brief description of the drawings

Further details and advantages of the invention are described in the following with reference to the attached drawings, which show in:

Figure 1: a schematic illustration of a sensor device according to a preferred embodiment of the invention;

Figure 2: a graphical illustration of a positive frequency shift measured with a sensor device according to Figure 1;

Figure 3: an illustration of a catalytic replacement scheme according to a preferred embodiment of the invention;

Figure 4: further details of the catalytic replacement scheme according to Figure 3;

Figures 5 to 7: graphical illustrations of practical results obtained with the invention; and

Figure 8: an illustration of a single replacement scheme according to a further embodiment of the invention.

Preferred embodiments of the invention

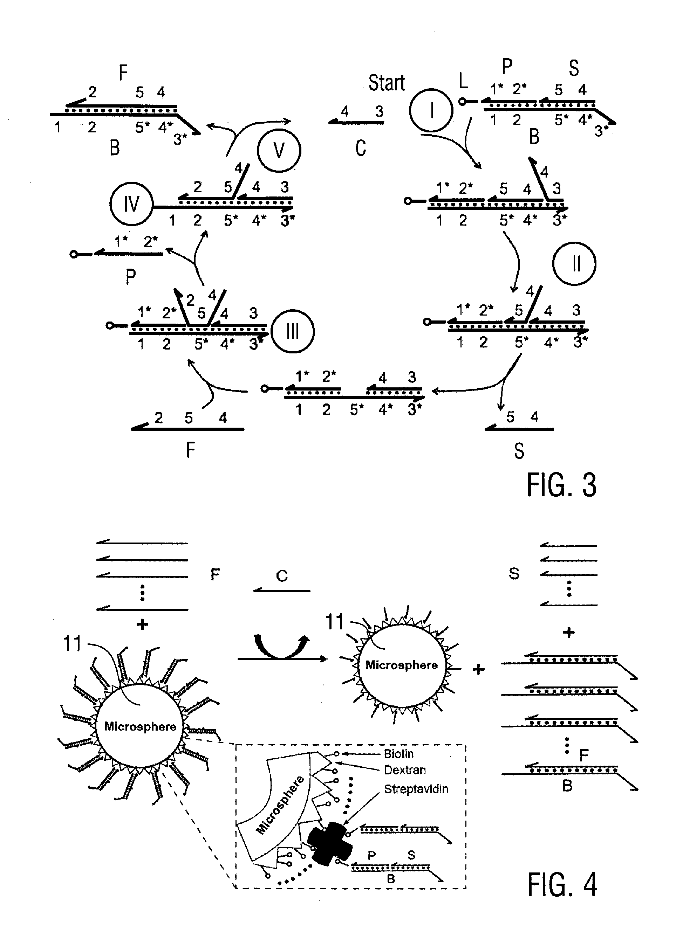

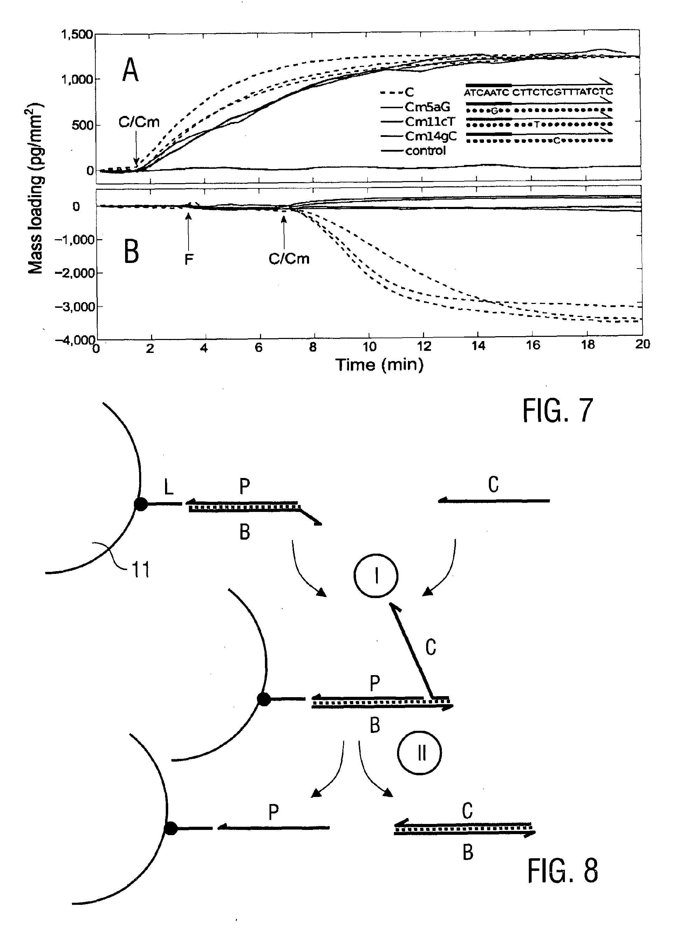

Preferred embodiments of the invention are described in the following with reference to the design of a sensor device configured for detecting target nucleic acid sequences according to the invention and with reference to the concept of strand displacement reactions used for detecting target nucleic acid sequences. Details of operating a laser source, detecting resonator light and measuring light wavelengths/ frequencies, and providing samples, in particular preparing nucleic acid sequences, are not described as far as they are known as such from prior art. Detailed reference is made to the catalytic replacement scheme of a strand displacement reaction, which represents a preferred embodiment of the invention (Figure 3). However, implementing the invention is not restricted to this scheme but also possible with the single replacement scheme described below (Figure 8). Exemplary reference is made to biotin-streptavidin linkers functionalizing the WGM resonator of the sensor device. The invention is not restricted to the use of these linkers but also possible with other linker molecules, like e. g. heterobifunctional linkers, such those based on amino and carboxy groups activated by EDC/NHS Esters, homobifunctional linkers, such as those based on thiol groups. Furthermore, instead of physisorbing dextran to immoblize the linker groups on the microsphere surface, the glass microsphere may also be covalently modified with silane agents to introduce functional groups, e.g. using silanes which carry the amine linker or other linker groups. Finally, practical selection of double-strand precursor compounds and fuel molecules can be done by the skilled person in dependency on the hybridization properties of the target molecule to be detected.

Sensor device

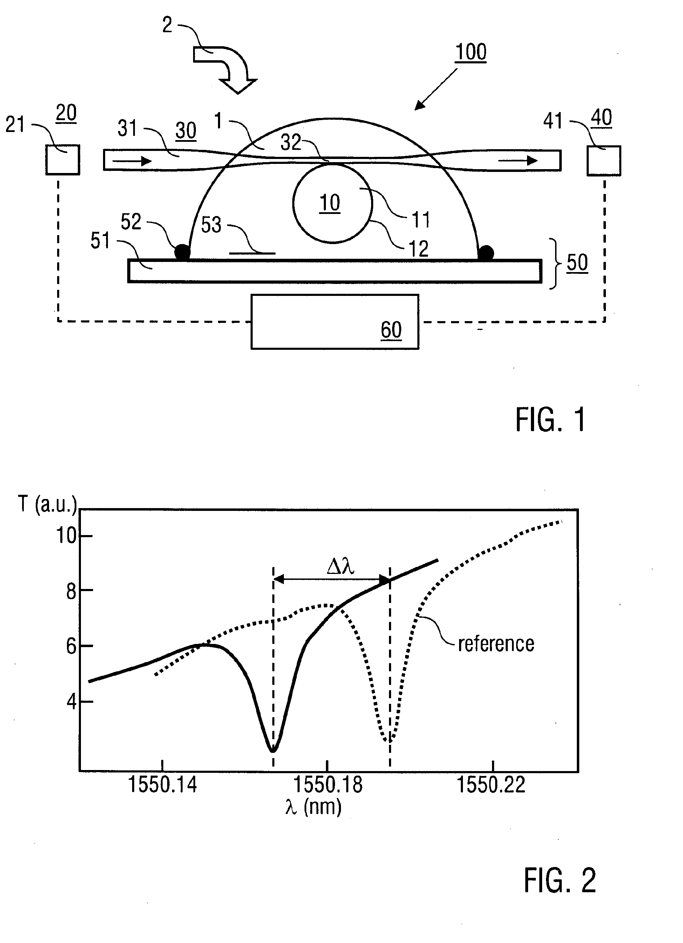

Preferred features of an inventive sensor devices are described in the following with reference to Figures 1 and 2. The sensor device 100 comprises an optical WGM resonator 10, a light source device 20, an optical waveguide 30, a detector device 40, a sample cell 50 and a control device 60.

The WGM resonator 10 comprises a microsphere 11 having a functionalized surface 12. The microsphere 11 is made of silica, and it is fabricated e. g. by melting the tip of a short piece of optical fiber (e. g. SMF-28) with an oxygen-butane microtorch so that surface tension in the melted glass tip forms the silica microsphere having a 300 $\mu$m to 400 $\mu$m diameter. The microsphere-on-a-stem is mounted on a microstage (not shown) for controlled coupling to the optical waveguide 30. For specific DNA detection, the surface 12 of the microsphere 11 is functionalized with biorecognition elements, like e. g. a 22mer DNA oligonucleotide (see below, Figures 3 and 4).

The light source device 20 includes a laser source 21, preferably a continous wave (cw) laser, like e. g. a tunable distributed feedback laser (DFB) laser operating at a wavelength of about 1550 nm. The laser source 21 can be scanned through a wavelength range including the resonance frequency of the WGM resonator 10, e. g. every millisecond to obtain a transmission spectrum of the WGM resonator 10 with a spectral width of about 0.2 nm.

Sample light (excitation light) from the laser source 21 is coupled via an imaging optic (e.g. a lens optic, not shown) into the optical waveguide 30, which comprises an optical fibre 31 with a tapered fiber region 31. The taper is fabricated e. g. from a single mode SMF-28 fiber using a microtorch to heat and at the same time pull apart the fiber. The microsphere 11 and the tapered fiber region 31 are arranged such that sample light can be coupled into the microsphere 11 by evanescent coupling.

The detector device 40 is arranged for detecting light output from the optical fibre 31 of the optical waveguide 30. The light output includes sample light and resonator light evanescently coupled out of the microsphere 11. The detector device 40 includes e. g. a photodiode 41. Spectra are recorded and processed using e. g. a commercial software, like Lab-VIEW. With a practical example, transmission spectra of the WGM resonator 10 are acquired by the photodiode 41 in real time while rapidly sweeping the laser source 41 wavelength by about 0.2 nm.

The sample cell 50 comprises a substrate 51, like a glass slide, and a frame 52, e. g. made of an O ring. The frame 52 is glued to the substrate 51, and it has a height of e. g. 2 mm. Inside the sample cell 50, a miniature magnetic stir bar 53 is placed to homogenize the reactions. A droplet 1 of sample liquid 2 to be investigated is accommodated in the sample cell 50 by capillary forces. The droplet 1 has a volume of e. g. about 400 $\mu$l. After providing the droplet 1, e. g. using a pipette, the fiber-coupled microsphere 11 is immersed in the sample liquid.

The control device 60 is arranged for controlling the light source device 20 and the detector device 40 and for processing the spectra collected with the detector device 40. Optionally, the microstage of the microsphere 11, the magnetic stir bar 53 and/or further operation components of the sensor device 100 can be controlled as well. Furthermore, the control device 60 is adapted for analyzing the spectra collected with the detector device 40 and for providing an output indicating the detection of a target molecule. The control device 60 comprises e. g. a computer unit.

For implementing the inventive sensing method for detecting target nucleic acid sequences, the WGM resonator 10 is functionalized with a double-strand precursor compound and arranged in the sample cell 50. With a practical example of a DNA detection according to the invention, the double-strand precursor compound comprises single-stranded DNA oligonucleotide probes which are attached via biotin streptavidin linkers to a dextran hydrogel that is coated onto the silica microsphere by physisorption (see [13]). Commercially available DNA oligonucleotides and standard procedures for purifying them can be used.

With more details, the microsphere 11 is functionalized e. g. with the following procedure. The microsphere 11 is cleaned in an air oxygen plasma for 5 minutes immediately after fabrication, then immersed in a 2 $\mu$l hanging drop of a dextranbiotin solution (10 mg/ml, Life Technologies) until almost dry. After a brief rinse in water for 5 min, it is then incubated until almost dry in a hanging drop of 2 $\mu$l of 8 $\mu$M solution of biotinylated DNA oligonucleotides (C* or P, see table below), coupled to streptavidin at a molar ratio of about 2:1. After incubation, the sphere is rinsed again in the water and stored there until use. The dextran hydrogel coating is preferred for functionalizing the WGM resonator 10 since it prevents unspecific binding of DNA and supports a high surface density of attached biotin molecules that link to biotinlyated oligonucleotides via streptavidin. A surface density of e. g. about $10^{13}$/cm$^2$ for biotin-streptavidinlinked DNA oligonucleotides can be obtained.

In an initial phase of the inventive sensing method, the sample cell 50 includes a sample-free liquid, like water. The transmission spectrum of the WGM resonator 10 is measured as a reference. Subsequently, the sample liquid to be investigated is contacted with the WGM resonator, e. g. by supplying to the sample cell 50. The hybridization or dissociation of oligonucleotides with partial or full complementarity to these probes induces a mass change which can be observed from resonance wavelength shift. Changes of transmission spectrum resulting from unloading the precursor compound from the surface 12 of the microsphere 11 are monitored. An increasing resonance frequency shows the presence of the target molecule in the sample liquid.

Typical spectra of the microsphere 11, here before (dotted line, reference) and after (solid line) the unloading of precursor compound from the surface 12 of the microsphere 11 are shown in Figure 2. The resonance wavelength shift of the sensor device 100 (Figure 1), for example upon DNA hybridization, is quantitated in grams of nucleic acid mass loading per millimeter-squared sensor area, pg/mm$^2$ (see [24], [25]):

$$massloading = \frac{\Delta\lambda\,(n_s^2 - n_m^2)R}{\lambda\,2\,n_m \cdot dn/dc}$$

where $\Delta\lambda$ is the shift of resonance wavelength, $\lambda$ is the nominal wavelength of the laser source 21, $n_s$ = 1.46 and $n_m$ = 1.33 are the refractive indices of microsphere 11 and aqueous medium in the droplet 1, respectively, R is the approximate radius of the microsphere 11 as determined by microscopic imaging, and $dn/dc \sim 0.17 * 10^{-9}$ [mm$^3$/pg] is the approximate incremental refractive index change of a DNA solution (see [13]).

Catalytic replacement scheme of strand displacement reaction

For illustrating the catalytic replacement scheme according to a preferred embodiment of the invention, reference is made to Figures 3 and 4. Start step (step I) of Figure 3 schematically shows the double-strand precursor compound SBP with a primary strand B and first and second secondary strands P, S being hybridized with the primary strand B, a target nucleic acid sequence C (analyte C) to be detected, and a fuel strand F (single-strand precursor molecule). The first secondary strand P is connected via a biotin molecule L with the microsphere 11 (see Figure 4, insert).

Examples of the molecules used for practically testing the inventive device are summarized in the following table:

| Domain | Sequences | Length (nt) |
|---|---|---|
| C | 5'-ATCAATC CTTCTCGTTTATCTC-3' | 22 |
| C* | 5'-biotin-GAGATA AACGAGAAG GATTGAT-3' | 22 |
| S | 5'-CTTCTCGTTTATCTCCTGTA-3' | 20 |
| B | 5'-GCGATG GGTAAGAACTTTAGTG TACAG GAGATAAACGAGAAG GATTGAT-3' | 49 |
| F | 5'-CTTCTCGTTTATCTC CTGTA CACTAAAGTTCTTACC-3' | 36 |
| P | 5'-CACTAAAGTTCTTACC CATCG-biotin-3' | 21 |
| Cm5aG | 5'-ATCAGTC CTTCTCGTTTATCTC-3' | 22 |
| Cm11cT | 5'-ATCAATC CTTTTCGTTTATCTC-3' | 22 |
| Cm14gC | 5'-ATCAATC CTTCTCCTTTATCTC-3' | 22 |
| Fn | 5'-CTGTA CACTAAAGTTCTTACC-3' | 21 |
| C2 | 5'-TGTAACAGCAACTCCATGTGGA-3' | 22 |
| S2 | 5'-GCAACTCCATGTGGACTGTA-3' | 20 |
| B2 | 5'-GCGATG GGTAAGAACTTTAGTG TACAG TCCA-CATGGAGTTGC TGTTACA-3' | 49 |
| F2 | 5'-GCAACTCCATGTGGA CTGTA CACTAAAGTTCTTACC-3' | 36 |
| T | 5'-CCCTATAGTGAGTCGTATTAAT-3' | 22 |
| T* | 5'-biotin-ATTAATACGACTCACTATAGGG-3' | 22 |

The two precursor molecules, F and SBP, are selected based on the sequence of the target nucleic acid sequence C to be detected.

In the absence of C, SBP is double-stranded everywhere it is complementary to F, so no significant reaction occurs, and the two species are metastable (see Figure 4, left side). In the presence of the target nucleic acid sequence C, the second secondary strand S and the primary strand B are delocalized from P and the surface of the microsphere 11 as follows. The target nucleic acid sequence C acts catalytically to enable the reaction between SBP and F, resulting in the release of S and B from P. Domains (displayed as numbers in Figure 3) represent continuous subsequences of nucleic acids that act as a unit in hybridization and dissociation; dotted domains are complementary to non-dotted domains.

The second secondary strand S is replaced by target nucleic acid sequence C (step II), wherein S is released into the surrounding liquid. Subsequently, the fuel strand F replaces the first secondary strand S (step III), so that the primary strand B with the fuel strand F and the target nucleic acid sequence C are decoupled from the microsphere 11 (step IV). Finally, the fuel strand F completely replaces the target nucleic acid sequence C from the primary strand B, so that the fuel strand F hybridized with the primary strand B is released into the surrounding liquid and the target nucleic acid sequence C is released for further unloading steps (step V, see also Figure 4, right side)). At the end of the reaction cycle, P remains as a single-stranded product, and C is released to enable multiple turnover.

With the catalytic replacement scheme of Figures 3/4, the sensitivity of the sensor device 100 is improved at the molecular level, wherein each molecule of the detection target effects the release of multiple molecules from multi-stranded precursor complexes. Previous characterizations of similar catalysis systems (see [21], [29]) reported catalytic speedup of over 104 and maximum turnover of about 100. Using polyacrylamide gel electrophoresis (PAGE), the inventors have verified that the specific sequences used for the inventive integrated WGM system behaves qualitatively similarly in bulk solution.

The size of S and B are independent of that of C; designs that utilize larger S and B molecules could thus result in higher mass unloading and provide improved sensitivity. In tested designs, S is 20 nucleotides (nt) and B is 49 nt, compared to C being 22 nt (see table). Thus, even without catalytic turnover (e. g., see Figure 8), a 3-fold improvement in sensitivity due to the decoupling of the unloading mass to the analyte mass is expected.

<u>Results of practical tests</u>

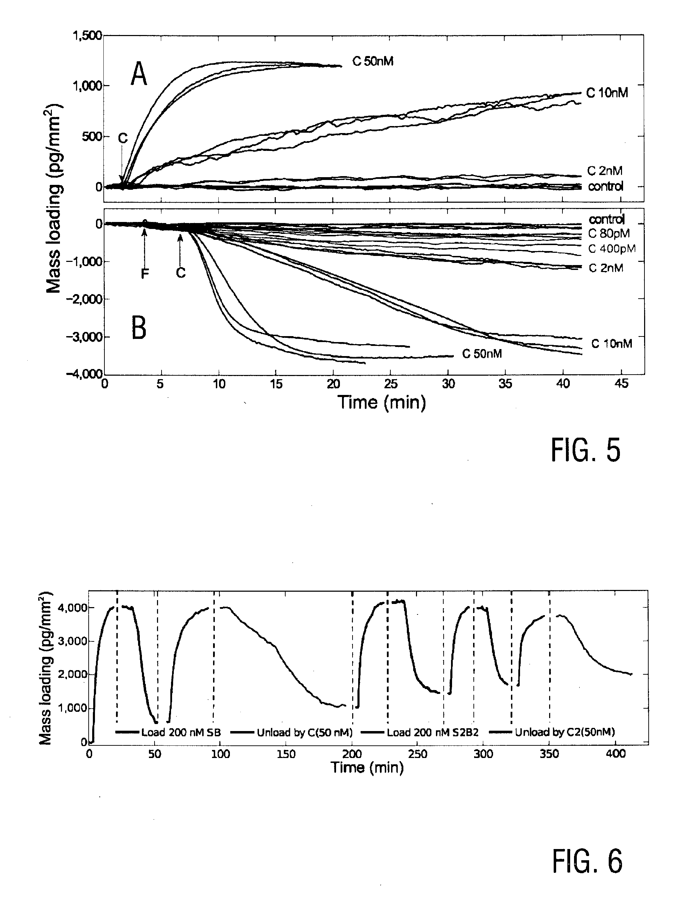

Figures 5A and 5B illustrate a comparison of molecular sensitivity of a conventional WGM sensor versus the inventive sensor device. According to Figure 5A, with the conventional WGM sensor, a direct hybridization of target C onto the probe C* has been measured, wherein the probe C* was preattached to the microsphere surface. 4 $\mu$l of target sequence C was injected at t = 200 s into the sample cell with 400 $\mu$l buffer to achieve final C concentrations of 50 nM, 10 nM or 2 nM, resp.. Control experiments were done by immersing the sensors in the sample cell without injecting anything. Figure 5B shows a test of the inventive sensor device 100 in which target C catalytically unloads strands S and B (see Figures 3, 4). 4 $\mu$l of F was injected at t = 200 s into the sample cell with 400 $\mu$l buffer to achieve final F concentration of 400 nM. At t = 400 s, 4 $\mu$l of C was injected to achieve final concentrations of 50 nM, 10 nM, 2 nM, 400 pM or 80 pM. The control trace shows the behavior of the system in the absence of C.

The results of Figure 5A in terms of sensor response and timescales are consistent with previous DNA detection schemes using label-free WGM biosensors and direct hybridization (see [13], [15], [17], and [26]). While the hybridization reaction could be kinetically limited, potentially by diffusion, as a benchmark for comparison against the inventive device, 40 minutes were selected as the allowed reaction time to compare molecular sensitivity.

Figure 5B shows the experimental results of the inventive sensor device, in which C catalytically unloads molecules from the microsphere surface. With the inventive sensor device, 400 pM concentration of analyte C produces a mass unloading signal of about 600 pg/mm2 within 40 minutes, six times as high as the mass loading yielded by 2 nM of C in the conventional hybridization-based WGM sensor. Consequently, a 30-fold improved sensitivity can be expected. Experimentally, a reliable detection of ~80 pM for 22-mer oligonucleotides has been shown, corresponding to at least 25-fold sensitivity improvement over the conventional WGM biosensor. At 80pM concentration, there is less than 32 fmol of the DNA analyte in the droplet cell, setting a new sensitivity record for label-free microcavity biosensors (see [1], [11]).

The total mass loading for the conventional direct hybridization of target C is about 1200 pg/mm2 (Figure 5A), while the total mass unloading for the inventive WGM catalytic network mechanism is about 3500 pg/mm2 (Figure 5B). This shows that the WGM signal is indeed proportional to the length of oligonucleotides: the loaded sequence C is 22 nt long, and the unloaded sequences S and B are 20 and 49 nt long, respectively. This also confirms that the inventive WGM sensor device is reproducibly modified with biotin-streptavidin linked oligonucleotides at surface concentrations of about 1011 strands/mm$^2$, consistent with previous observations for the dextran surface functionalization technique (see [13]).

Combining the 30-fold expected sensitivity improvement with the 3-fold increased mass unloading, it is expected that each molecule of C on average yielded 10 rounds of reaction turnover over the course of the 40 minutes of observed reaction. This can be quantitatively confirmed by designing an alternative version of F that does not allow multiple turnover. In this case, the kinetics of mass unloading is slowed significantly and the molecular sensitivity decreases approximately 10-fold.

Figure 6 illustrates repeated reuse of the inventive sensor device to detect multiple different target sequences. As shown, the same physical sensor was demonstrated to alternatingly detect 2 different nucleic acid sequences through 5 cycles of detection, showcasing both its reusability and its versatility. Loading experiments were performed with 200 nM of SB/S2B2 and unloading experiments were performed with 400 nM F/F2 and 50 nM C/C2. The mass loadings were converted from the relative shifts of each loading/unloading cycle and offset so that the next loading/unloading cycle starts where the last one ended.

The tests of Figure 6 show that limitations in terms of versatility and reusability can be overcome with the inventive sensor device. The inventors have tested that microspheres functionalized with the same DNA sequence (P) could be used for the versatile detection of different analytes. This is feasible for the inventive sensor device because the sequence of P is independent of the sequence of the detection target C. Furthermore, it has been shown, that the same WGM microsphere can be reused. At the end of a detection reaction, the products S and FB and the analyte C are delocalized from the microsphere surface; consequently, through a mild buffer exchange the WGM can be restored to allow repeated detection cycles. Figure 6 shows that the same WGM microsphere (functionalized with the same P molecules) can be used through 5 distinct cycles of detection to alternating detect 2 different sequences.

Figure 7 illustrates a comparison of single nucleotide polymorphism (SNP) specificity of a conventional WGM sensor versus the inventive sensor device. Figure 7A shows the SNP detection with the conventional hybridization method. The target (dashed lines) and three SNP variants (drawn lines) were injected at t = 100 sec at 50 nM concentration. The inset shows the sequences of the target and the SNP variants; the thicker line segment denotes the toehold region. Figure 7B shows the SNP detection with the inventive sensor device. Precursor F with a final concentration of 400 nM was injected at t = 200 s and followed by the injection of target or SNP variant (C and Cms, respectively) at t = 400 s to a concentration of 50 nM. Again, the target is shown with dashed lines, while the three SNP variants are shown with drawn lines.

According to Figure 7A, the conventional sensor was tested with 3 SNP variants of the intended analyte C (Cm5aG, Cm11cT, and Cm14gC). The three single base changes (A to G at position 5, C to T at position 11, G to C at position 14), were selected to be representative of the variety of both positions along the analyte sequence and of the thermodynamics of single-base changes. These SNP variants induced a similar kinetics and total amount of mass loading as the analyte C, so the standard WGM sensor is not specific to SNPs. On the contrary, according to Figure 7B, the inventive sensor device challenged by the same SNP variants is capable to sense significantly lower mass unloading by the SNP variants compared with that of the intended analyte C.

Single replacement scheme of strand displacement reaction

For illustrating the single replacement scheme according to a further embodiment of the invention, reference is made to Figure 8. Start step (step I) of Figure 8 schematically shows the double-strand precursor compound BP with a primary strand B and a secondary strands P being hybridized with the primary strand B, and a target nucleic acid sequence C (analyte C) to be detected. The secondary strand P is connected via a biotin molecule L with the microsphere 11.

As with the scheme of Figure 3, in the absence of C, BP is stably double-stranded. In the presence of the target nucleic acid sequence C, the primary strand B is delocalized from P and the surface of the microsphere 11. The secondary strand P is replaced by target nucleic acid sequence C (step I), wherein the target nucleic acid sequence C hybridized with the primary strand B is released into the surrounding liquid, while P remains as a single-stranded product at the microsphere 11 (step II). Accordingly, with the single replacement scheme, unloading of the microsphere 11 and increasing the resonance frequency thereof can be obtained as with the catalytic replacement scheme.

The features of the invention disclosed in the above description, the drawings and the claims can be of significance both individually as well as in combination for the realization of the invention in its various embodiments.

**Claims**

**1.**   Sensor device (100), which is adapted for detecting target molecules having a target nucleic acid sequence, comprising:

-   an optical whispering gallery mode (WGM) resonator (10) having a resonance frequency, wherein
-   the WGM resonator is functionalized with a double-strand DNA precursor compound and the resonance frequency depends on a mass load provided by the double-strand precursor compound,
- the double-strand precursor compound is capable of a target-specific strand displacement reaction with the target molecules, and
- in response to the strand displacement reaction, the double-strand precursor compound is capable to be partially decoupled from the WGM resonator (10), wherein the mass load can be decreased and the resonance frequency of the WGM resonator can be increased.

2. Sensor device according to claim 1, wherein

- the double-strand precursor compound has a primary strand and a secondary strand being hybridized with the primary strand, wherein the secondary strand is connected with the WGM resonator (10), and
- the double-strand precursor compound is capable to be partially decoupled from the WGM resonator (10) by replacing the secondary strand by the target molecule and dissociating the primary strand with the target molecule from the WGM resonator (10).

3. Sensor device according to claim 1, wherein

- the double-strand precursor compound (SBP) has a primary strand (B), a first secondary strand (P) being hybridized with the primary strand (B) and a second secondary strand (S) being hybridized with the primary strand (B), wherein
- the first secondary strand (P) is connected with the WGM resonator (10), and
- the double-strand precursor compound (SBP) is capable to replace the second secondary strand (S) by the target molecule (C), and
- the double-strand precursor compound (SBP) is capable to replace the first secondary strand (P) by a further single-strand precursor compound (F) and to dissociate the primary strand (B) hybridized with the target molecule (C) and the single-strand precursor compound (F) from the WGM resonator (10).

4. Sensor device according to claim 3, wherein

- the single-strand precursor molecule (F) is capable to replace the target molecule from the primary strand, so that the target molecule is released.

5. Sensor device according to one of the foregoing claims,

- the WGM resonator (10) is provided with at least one optical waveguide (30) or optical prism being arranged for at least one of in-coupling sample light into the WGM resonator (10) and out-coupling resonator light out of the WGM resonator (10).

6. Sensor device according to claim 5, further comprising

- a continuous wave tunable laser source (21) being arranged for generating the sample light.

7. Sensor device according to one of the foregoing claims, further comprising

- a sample cell (50) being arranged for accommodating the WGM resonator and a sample liquid to be investigated.

8. Sensor device according to one of the foregoing claims, wherein

- the WGM resonator (10) has a surface layer made of a biotin streptavidin compound to which the double-strand precursor compound is coupled.

9. Sensing method for detecting target molecules including nucleic acid sequences,

- providing an optical whispering gallery mode (WGM) resonator (10) having a resonance frequency, wherein the WGM resonator (10) is functionalized with a double-strand precursor compound and the resonance frequency depends on a mass load provided by the double-strand precursor compound, wherein
- the double-strand precursor compound is capable of a strand displacement reaction with the target molecules, and

- the double-strand precursor compound is capable to be partially decoupled from the WGM resonator (10), and the mass load can be decreased and the resonance frequency of the WGM resonator can be increased in response to the strand displacement reaction,

said method comprising the steps of:

- contacting a sample liquid to be investigated with the WGM resonator (10),
- monitoring the resonance frequency of the WGM resonator (10), and
- detecting the target molecules if the resonance frequency of the WGM resonator (10) is increased.

10. Sensing method according to claim 9, wherein

- the double-strand precursor compound has a primary strand and a secondary strand being hybridized with the primary strand, wherein the secondary strand is connected with the WGM resonator (10), wherein
- the strand displacement reaction comprises partially decoupling the double-strand precursor compound from the WGM resonator (10) by replacing the secondary strand by the target molecule and dissociating the primary strand with the target molecule from the WGM resonator (10).

11. Sensing method according to claim 9, wherein

- the double-strand precursor compound (SBP) has a primary strand (B), a first secondary strand (P) being hybridized with the primary strand (B) and a second secondary strand (S) being hybridized with the primary strand (B), wherein the first secondary strand (P) is connected with the WGM resonator (10), wherein the strand displacement reaction comprises
- a first step of replacing the second secondary strand (S) by the target molecule (C), and
- a second step of replacing the first secondary strand (P) by a further single-strand precursor molecule (F) and dissociating the primary strand (B) hybridized with the target molecule (C) and the single-strand precursor compound (F) from the WGM resonator (10).

12. Sensing method according to claim 11, wherein the strand displacement reaction comprises

- a third step of replacing the target molecule from the primary strand, so that the target molecule is released.

FIG. 1

FIG. 2

FIG. 3

FIG. 4

FIG. 5

FIG. 6

FIG. 7

FIG. 8

Europäisches
Patentamt

European
Patent Office

Office européen
des brevets

# EUROPEAN SEARCH REPORT

Application Number

EP 13 00 3900

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FRANK VOLLMER: "Enhancing whispering gallery mode biosensing", 2013 15TH INTERNATIONAL CONFERENCE ON TRANSPARENT OPTICAL NETWORKS (ICTON), 1 June 2013 (2013-06-01), pages 1-1, XP055096983, DOI: 10.1109/ICTON.2013.6602810 ISBN: 978-1-47-990683-3 | 1-4,9 | INV.<br>G01N21/77<br>C12Q1/68 |
| Y | * abstract * | 5-8, 10-12 | |
| Y | FRANK VOLLMER ET AL: "Review Label-free detection with high-Q microcavities: a review of biosensing mechanisms for integrated devices", NANOPHOTONICS, vol. 1, no. 3-4, 1 January 2012 (2012-01-01), XP055097213, ISSN: 2192-8606, DOI: 10.1515/nanoph-2012-0021 | 5-8 | |
| A | * the whole document * | 1,2,9 | TECHNICAL FIELDS SEARCHED (IPC) |
| Y,D | ZHANG DAVID YU ET AL: "Optimizing the specificity of nucleic acid hybridization", NATURE CHEMISTRY, NATURE PUBLISHING GROUP, UK, vol. 4, no. 3, 1 March 2012 (2012-03-01), pages 208-214, XP008165567, ISSN: 1755-4349, DOI: 10.1038/NCHEM.1246 [retrieved on 2012-01-22] * the whole document * | 10-12 | C12Q<br>G01N |

-/--

The present search report has been drawn up for all claims

| Place of search | Date of completion of the search | Examiner |
|---|---|---|
| Munich | 21 January 2014 | Weinberger, Thorsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 13 00 3900

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PENG YIN ET AL: "Programming biomolecular self-assembly pathways", NATURE, NATURE PUBLISHING GROUP, UNITED KINGDOM, vol. 451, no. 7176, 17 January 2008 (2008-01-17), pages 318-322, XP002661061, ISSN: 0028-0836, DOI: 10.1038/NATURE06451 * the whole document * | 10-12 | |
| Y | B. LI ET AL: "Rational, modular adaptation of enzyme-free DNA circuits to multiple detection methods", NUCLEIC ACIDS RESEARCH, vol. 39, no. 16, 1 September 2011 (2011-09-01), pages e110-e110, XP055097100, ISSN: 0305-1048, DOI: 10.1093/nar/gkr504 * the whole document * | 10-12 | |
| Y | DAVID YU ZHANG ET AL: "Engineering entropy-driven reactions and networks catalyzed by DNA", SCIENCE, AMERICAN ASSOCIATION FOR THE ADVANCEMENT OF SCIENCE, WASHINGTON, DC; US, vol. 318, no. 5853, 16 November 2007 (2007-11-16), pages 1121-1125, XP002715513, ISSN: 0036-8075, DOI: 10.1126/SCIENCE.1148532 * the whole document * | 10-12 | TECHNICAL FIELDS SEARCHED (IPC) |

The present search report has been drawn up for all claims

| Place of search | Date of completion of the search | Examiner |
|---|---|---|
| Munich | 21 January 2014 | Weinberger, Thorsten |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VOLLMER, F. et al.** *Nanophotonics,* 2012, vol. 1, 267-291
- **FAN, X. D. et al.** *Anal. Chim. Acta,* 2008, vol. 620, 8-26
- **QAVI, A. J. et al.** *Anal. Bioanal. Chem.,* 2009, vol. 394, 121-135
- **HUNT, H. K. et al.** *Nanoscale,* 2010, vol. 2, 1544-1559
- **VOLLMER, F. et al.** *Nat. Methods,* 2008, vol. 5, 591-596
- **YOSHIE, T. et al.** *Sensors,* 2011, vol. 11, 1972-1991
- **LIN, S. Y. et al.** *Lab Chip,* 2011, vol. 11, 4047-4051
- **LU, T. et al.** *Proc. Natl. Acad. Sci. U. S. A.,* 2011, vol. 108, 5976-5979
- **LOPEZ-YGLESIAS, X. et al.** *J. Appl. Phys,* 2012, 111
- **VOLLMER, F. et al.** *Proc. Natl. Acad. Sci. U. S. A.,* 2008, vol. 105, 20701-20704
- **QAVI, A. J. et al.** *Angew. Chem.-Int. Edit.,* 2010, vol. 49, 4608-4611
- **NAKATANI, K. et al.** *Chembiochem,* 2004, vol. 5, 1623-1633
- **VOLLMER, F. et al.** *Biophys. J.,* 2003, vol. 85, 1974-1979
- **QAVI, A. J. et al.** *Anal. Chem.,* 2011, vol. 83, 6827-6833
- **SUTER, J. D. et al.** *Biosens. Bioelectron.,* 2008, vol. 23, 1003-1009
- **ZHU, J. G. et al.** *Nat. Photonics,* 2010, vol. 4, 46
- **SCHELER, O. et al.** *Biosens. Bioelectron.,* 2012, vol. 36, 56-61
- **ZHANG, D. Y. et al.** *Nat. Chem.,* 2012, vol. 4, 208-214
- **YIN, P. et al.** *Nature,* 2008, vol. 451, 318-U314
- **LI, B. L. et al.** *Nucleic Acids Res.,* 2011, 39
- **ZHANG, D. Y. et al.** *Science,* 2007, vol. 318, 1121-1125
- **ZHANG, D.Y. et al.** *Nat. Chem.,* 2011, vol. 3, 103-113
- **ZHANG, D.Y. et al.** *J. Am. Chem. Soc.,* 2009, vol. 131, 17303-17314
- **BAASKE, M. et al.** *ChemPhysChem,* 2012, vol. 13, 427-436
- **ARNOLD, S. et al.** *Optics Letters,* 2003, vol. 28, 272-274
- **SUTER, J.D. et al.** *Biosens. Bioelectron.,* 2010, vol. 26, 1016-1020
- **LEE, M. et al.** *Anal. Biochem.,* 2000, vol. 282, 142-146
- **ZUKER, M.** *Nucleic Acids Res.,* 2003, vol. 31, 3406-3415
- **ZHANG, D. Y. et al.** *Nucleic Acids Res.,* 2010, vol. 38, 4182-4197